# EUROPEAN PATENT APPLICATION

(11) **EP 0 788 775 A1**
(43) Date of publication of application: **13.08.1997**
(21) Application number: 96201735.6
(22) Date of filing: 21.06.1996
(51) Int. Cl.: A61B 17/32

(54) **Single-use surgical scalpel**

(30) Priority: 24.10.1995 IE 950829
(71) Applicant: Boyle, Francis, Balinfull, County Sligo (IE)
(72) Inventor: Boyle, Francis, Balinfull, County Sligo (IE)
(74) Representative: Boyce, Conor

(57) **Abstract**

A single-use disposable surgical scalpel comprises a handle (1) having a narrow chamber (3) open along one edge of the handle and a surgical blade (5) pivotally mounted on the handle. The blade (5) is rotatable from a cutting position in which the blade (5) projects forwardly of the handle (1) to a disposal position in which substantially the entire blade is accommodated within the chamber (3). The blade (5) is an interference fit in the chamber 3, so that the blade is effectively prevented from being moved out of the disposal position.

## Description

The present invention relates to a disposable scalpel and more particularly to a single-use surgical scalpel.

Surgical scalpels are normally used only once, mainly due to the high risk or contamination from body fluids from which disease can be transmitted. Single-use scalpels are well known. In one group of known scalpels, a guard is provided for the scalpel blade when it is not in use. To ensure the blade is used only once, a locking arrangement must be included to secure the guard to the scalpel. In another group of scalpels, the scalpel blade can be moved between a cutting position and a storage position within the handle. The blade is arranged on a carrier which is slidably mounted within the scalpel handle. Initially, the blade is provided in a retracted position for shipment from which the blade may be urged forward to be exposed for use. After use, the blade is fully retracted into the handle and a locking means is provided to lock the blade in that position for disposal.

The known arrangements have the disadvantage that they are relatively expensive to manufacture compared to standard disposable scalpels which do not have any safety features.

It is an object of the present invention to provide a single-use safety scalpel which may be manufactured inexpensively and by mass production.

This object is met by the invention claimed in claim 1.

Embodiments of the invention will now be described, by way of example only, with reference to the accompanying drawings, in which:
Figure 1 is a bottom plan view of a first embodiment of scalpel according to the invention in which the blade is in a cutting position;
Figure 2 is a side elevation of the scalpel of Figure 1 in which the blade is in a cutting position;
Figure 3 is a sectional side elevation taken on the line X-X in Figure 1 but showing the blade in its disposal position;
Figure 4 is a side elevation of a second embodiment of scalpel, omitting the handle cover member, in which the blade is shown in a cutting position;
Figure 5 is a side elevation of the scalpel of Figure 4 including the handle cover member;
Figure 6 illustrates the handle cover member of the scalpel of Figure 4;
Figure 7 is a bottom plan view of the scalpel of Figure 4 in which the blade is in the cutting position;
Figure 8 is a cross-section taken on the line W-W of Figure 5;
Figure 9 is a cross-section taken on the line W-W of a variation of the second embodiment of the invention; and
Figure 10 is a cross-section taken on the line X-X of Figure 9.

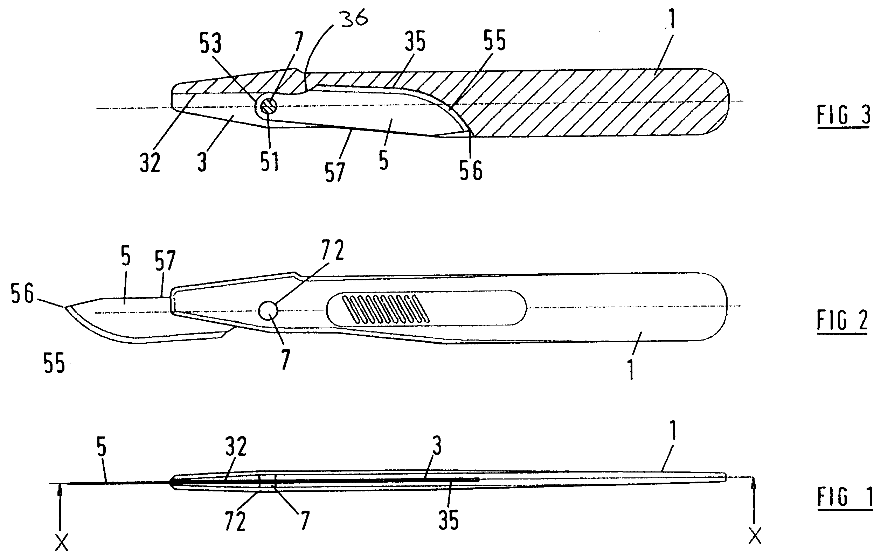

Referring first to Figures 1 to 3 of the drawings, a single-use safety scalpel comprises a handle 1 having a narrow chamber 3 open along one edge of the handle. A surgical blade 5 is mounted about a pivot pin 7 in the chamber. The surgical blade 5 is substantially similar to a standard blade except that it is provided with a pivot aperture 51 towards its rear end 53 to accommodate the pivot pin 7. The rear end 53 of the blade 5 has a rounded profile so that the blade 5 can be rotated about the pivot pin 7.

The scalpel is packaged and shipped with the blade 5 in the cutting position as shown in Figures 1 and 2. In this position, the blade 5 is held in line with the longitudinal axis of the handle 1 and extending forwardly therefrom. The blade 5 has a cutting edge 55 along one side and a blunt edge 57 along the other side which meet at a tip 56 at the forward end of the blade. In the cutting position the blunt edge 57 of the blade abuts the portion 32 of the inner edge 36 of the chamber 3 forwardly of the pivot pin 7 to provide support to the blade 5 when cutting.

The blade 5 is moved from the cutting position to a disposal position, Figure 3, by rotating the blade 5 by approximately 180 degrees about the pivot pin 7, so that the cutting edge 55 of the blade abuts the portion 35 of the inner edge 36 of the chamber 3 rearwardly of the pivot pin 7. In the disposal position substantially the entire blade 5 lies within the chamber 3, so that no part of it may be reached to extract the blade.

To move the blade 5 from the cutting position to the disposal position, a surgeon or other medical practitioner either forces the blunt edge 57 of the blade against an object such as a table to deflect the blade 5 or uses a finger against the blunt edge 57.

The rounded rear end 53 of the blade 5 and the inner edge 36 of the chamber are so profiled as to resist rotation of the blade out of the disposal position, so that a significant force is required to overcome the resistance to movement between the rounded rear end 53 of the blade and the inner edge 36.

Further resistance to the rotation of the blade 5 about the pivot pin 7 is provided by the interior walls of the chamber 3 which frictionally engage the opposite surfaces of the blade 5 in the disposal position such that the blade is an interference fit in the chamber. To this end the handle 1 is formed of a plastics material which when in contact with the blade provides a friction surface which is used to resist the movement of the blade.

The fact that the blade 5 is substantially entirely accommodated within the chamber 3 in the disposal position, together with the frictional force exerted on the blade by the interior walls of the chamber and the resistance to rotation provided by the rounded rear end 53 of the blade, effectively prevents the blade 5 from being withdrawn from the disposal position for re-use. Of course, the interference fit of the blade in the chamber, and the resistance to rotation provided by the rear end of the blade, will also resist movement of the blade from the cutting position into the disposal position. However, in that case the exposed blade provides a purchase permitting leverage of the blade into the chamber, as described above. No such purchase is available when the blade is in the disposal position.

The handle 1 of the scalpel is preferably formed in two halves, one half having a pivot pin 7 integrally formed thereon, on which the blade is placed, and the other half having a pin receiving aperture 72. The two halves are then bonded together, with the blade pivotably mounted to the pin 7.

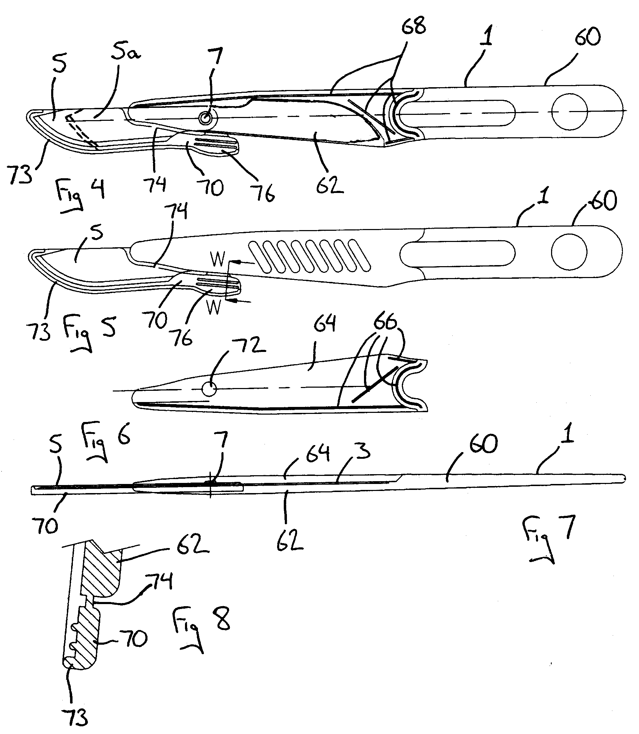

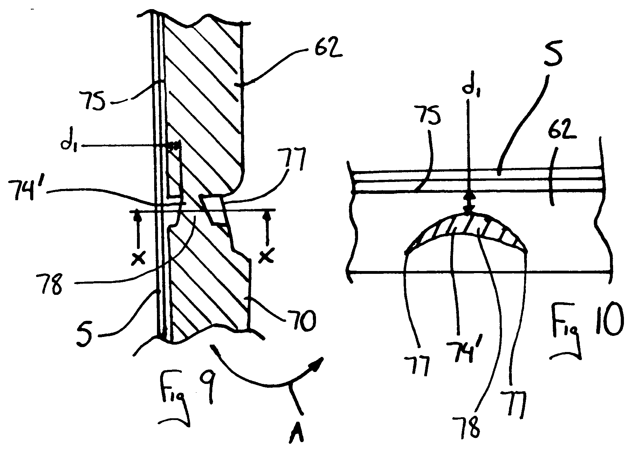

A second embodiment of the invention is shown in Figures 4 to 8, in which parts which are the same or equivalent to parts shown in Figures 1 to 3 have been given the same reference numerals.

In Figures 4 to 8 the handle 1 is formed of two parts, a main body part 60 having a front portion 62 of reduced thickness, and a cover member 64 which is fitted to the front portion 60 by bonding ribs 66 on the cover member 64 to complementary ribs 68 on the front portion 62. These ribs 66, 68 space the cover member 64 from the front portion 62 so as to define a narrow gap therebetween which forms the chamber 3. The front portion 62 has a pivot pin 7 integrally formed thereon, which is received in an aperture 72 in the cover member 64 when the latter is fitted to the front portion.

As before, the blade 5 is pivotally mounted on the pivot pin 7 for rotation from a cutting position (Figures 4, 5 and 7) to a disposal position where the blade 5 is accommodated in the chamber 3 as described for the first embodiment. Once rotated into the disposal position the blade 5 is effectively prevented from moving out of the disposal position by the same features of construction described with reference to Figures 1 to 3 and which will not be repeated here.

The scalpel of Figures 4 to 8 also has a tearaway blade cover 70 which protects the blade when the scalpel is shipped and which must be removed prior to use of the scalpel. The cover 70 is formed as an integral forward extension of the front portion 62 of the handle 1, and is present on one side of the blade 5 only (see especially Figure 8). The shape and size of the cover 70 are such as to embrace the full area of the blade 5 where it projects from the handle 1. The cover 70 lies closely against the surface of the blade 5 and has a lip 73 which extends laterally over the cutting edge 55. A single size/shape cover 70 may be used for a range of blade sizes, as indicated by the small blade 5a in Figure 4 which does not extend fully to the periphery of the cover 70.

The cover 70 is integrally joined to the front portion 62 of the handle by a line of weakness 74 constituted by a section of the plastics material whose thickness is substantially less than the thickness of the front portion 62 and the cover 70. A tab 76 is formed at the rear end of the cover 70, and by gripping this tab and pulling forwardly the material is readily torn along the line of weakness to detach the cover 70. Then, the scalpel may be used and disposed of in the manner described for the embodiment of Figures 1 to 3.

In a variation of the second embodiment, Figures 9 and 10, a line of weakness 74' is formed between the cover 70 and front portion 62 in a crescent shape with pointed ends 77 of the crescent facing away from the side 75 of the front portion 62 adjacent the blade 5. The crescent 74' is spaced a minimum distance d1 of approximately 0.5mm from the face 75 and increases in thickness towards the cover 70. The pointed ends 77 of the crescent are more easily compressed than its centre portion 78 and, as such, the cover 70 is more easily bent in the direction of the arrow A when being broken away from the blade 5 and the front portion 62.

The invention is not limited by or to the specific embodiments described which can undergo considerable variation without departing from the scope or spirit of the invention.

## Claims

1. A single-use surgical scalpel comprising a handle (1) having a narrow chamber (3) open along one edge of the handle and a surgical blade (5) pivotally mounted on the handle, the blade having a cutting edge (55) and a blunt edge (57) which meet at a tip (56) of the blade and being rotatable from a cutting position in which the blade (5) projects forwardly of the handle (1) to a disposal position in which at least the cutting edge (55) of the blade is accommodated within the chamber (3), the scalpel further including means to prevent the blade from being moved out of the disposal position.

2. A scalpel as claimed in claim 1, wherein the pivot point (7) of the blade (5) is inside the chamber (3), whereby in the cutting position the blunt edge (57) of the blade abuts the inner edge (36) of the chamber forwardly of the pivot point (7) to provide support to the blade when cutting.

3. A scalpel as claimed in claim 2, wherein substantially the entire blade (5) is accommodated within the chamber (3) in the disposal position.

4. A scalpel as claimed in claim 3, wherein the prevention means comprises the interior walls of the chamber (3) which frictionally engage the opposite surfaces of the blade (5) in the disposal position such that the blade is an interference fit in the chamber.

5. A scalpel as claimed in any preceding claim, wherein the prevention means comprises the rear end (53) of the blade (5) and the inner edge (36) of the chamber which cooperate to resist rotation of the blade from the disposal position.

6. A scalpel as claimed in any preceding claim, further including a tearaway blade cover (70) formed as an integral forward extension of the handle (1), the cover being present on only one side of the blade (5)and having a shape and size such as to embrace the full area of the blade, the cover (70) being integrally joined to the handle by a line of weakness (74) permitting ready detachment of the cover from the handle.
